# EUROPÄISCHE PATENTSCHRIFT

(11) **EP 1 249 216 B1**
(45) Veröffentlichungstag und Bekanntmachung des Hinweises auf die Patenterteilung: **15.10.2003**
(21) Anmeldenummer: 01109195.6
(22) Anmeldetag: 12.04.2001
(51) Int. Cl.: A61G 11/00, G01R 33/28

(54) **Inkubator für die Kernspintomographie**
Incubator for magnetic resonance
Incubateur pour résonance magnétique

(43) Veröffentlichungstag der Anmeldung: 16.10.2002
(73) Patentinhaber: Lönneker-Lammers Torsten, 22143 Hamburg (DE)
(72) Erfinder: Lönneker-Lammers Torsten, 22143 Hamburg (DE)
(74) Vertreter: Glawe, Delfs, Moll & Partner

(56) Entgegenhaltungen:
- EP-A- 0 864 295
- WO-A-98/48756
- US-A- 5 730 355
- US-A- 5 800 335
- US-A- 5 935 055

## Beschreibung

Die Erfindung betrifft einen Inkubator für neu- und frühgeborene Patienten, der in das Magnetfeld eines Kernspintomographen einführbar ist.

Früh- und Neugeborene sind häufig nicht in der Lage, ihre Körpertemperatur selbständig aufrecht zu erhalten. Daher werden sie in sog. Inkubatoren warm gehalten. Diese haben im allgemeinen eine beträchtliche Größe und enthalten Metallteile, insbesondere Eisenteile. Solche Inkubatoren können daher nicht in das Magnetfeld eines Kernspintomographen eingebracht werden, so daß es nicht, was an sich wünschenswert wäre, möglich ist, den Patienten mit Kernspintomographie zu untersuchen, während er sich im Inkubator befindet.

Es ist zwar ein Inkubator bekannt, mit dem der Patient in das Magnetfeld des Kernspintomographen eingebracht werden kann (DE 196 17 739 Cl). Dieser Inkubator ist aber verhältnismäßig einfach aufgebaut und weist z.B. keine Einrichtungen zum Untersuchen des Patienten, insbesondere keine Spule auf, mit der die Signale der magnetischen Resonanz aufgenommen werden können. Außerdem erfordert dieser Inkubator eine Druckfluidquelle zum Betrieb des Ejektors für die Luftumwälzung und externe Gasflaschen. Der Inkubator ist daher schwierig zu transportieren.

Ein Inkubator der eingangs genannten Art (WO 98/48756) weist zwar Einrichtungen zum Untersuchen des Patienten auf, z.B. eine Spule für die Signale der magnetischen Resonanz. Der Inkubator ist aber über mehrere Kabel und Schlauchleitungen mit einer entfernt vom Magnetfeld anzuordnenden Basiseinheit verbunden, ist daher wiederum auf nur sehr umständliche Weise zu transportieren. Soll eine Kernspinaufnahme vom Patienten gemacht werden, so müssen auf komplizierte Weise die Basiseinheit und der Inkubator zum Kernspintomographen gebracht werden.

*Ein bekannter Inkubator weist eine besonders ausgebildete Art der Luftströmung und Regelung dieser Strömung sowie der Temperatur auf (US-A-5,730,355). Dieser Inkubator ist aber offenbar nicht für die Einführung in einen Kernspintomographen ausgebildet, so daß Probleme wegen der Wechselwirkung mit dem Magnetfeld und der Meßelektronik eines Kernspintomographen nicht auftreten können.*

Wegen der Probleme, daß Elektromotoren und elektronische Schaltkreise einerseits durch das Magnetfeld in ihrer Funktion gestört werden könnten und andererseits Signale abgeben könnten, die die aufgenommenen Signale bei der Kernspintomographie stören, hat man es bisher für nötig gehalten, z.B. die elektrische Steuerung und den elektrischen Antrieb von Ventilatoren und dergleichen in einem getrennten Bauteil vorzusehen (EP 0 864 295 A2). Außerdem wurde wegen der notwendigen geringen Baugröße dieser Inkubatoren die Frage der gleichmäßigen Wärmeverteilung im Patientenraum bisher nicht zufriedenstellend gelöst. Bei einem der vorbekannten Inkubatoren (DE 196 17 739 C1) erfolgt zwar eine wirksame Umspülung des doppelwandig ausgebildeten Aufnahmeraumes für den Patienten. Durch den geringen Querschnitt ist jedoch bei Einsatz mit Patient eine gleichmäßige Wärme- und Strömungsverteilung nicht mehr gegeben, was zu erheblichen Problemen führen kann. Bei dem anderen vorbekannten Inkubator (WO 98/48756) ist überhaupt nicht ersichtlich, wie wirksam die Luftumwälzung bzw. -erneuerung sein soll und wie sie ausgebildet ist.

Die Aufgabe der Erfindung besteht in der Schaffung eines für die Kernspintomographie geeigneten Inkubators, der einfach zu transportieren ist und trotz kleiner Baugröße eine gleichmäßige Temperaturverteilung und somit wirksame und schonende Erwärmung/Warmhaltung des Patienten ermöglicht.

*Die erfindungsgemäße Lösung ist ein Inkubator* nach Anspruch 1. Erfindungsgemäß wurde also das Vorurteil überwunden, daß elektrisch angetriebene Einrichtungen nicht direkt am Inkubator angeordnet sein können. So ist z.B. nicht mehr der Antrieb des Ventilators durch ein Druckfluid erforderlich, das separat vom Inkubator erzeugt wird. Dieser Antrieb kann vielmehr elektrisch durch einen Motor erfolgen, der am Inkubator angebracht ist. Der Patientenraum wird dauernd mit Luft durchspült, die auf einer gesamten Längsseite einströmt und um die Längsachse des Inkubators eine Luftwalze bildet, in deren Achse die Luft an einem Ende des Patientenraums wieder abgesaugt wird.

Zweckmäßigerweise erfolgt die Luftzufuhr durch einen Kanal in einen Beruhigungsraum unterhalb der Liegefläche für den Patienten. Die Luft kann dann nach Überwinden eines Strömungswiderstandes zur Beschleunigung seitlich vom Patienten in den Patientenraum eintreten, so daß die erwähnte Luftbewegung auftritt. Da die Einrichtungen des Inkubators elektrisch angetrieben werden, muß zum Transport des Inkubators nur eine elektrische Verbindung gelöst und wieder hergestellt werden. Man könnte ohne weiteres einen Transportwagen für den Inkubator mit einem Akkumulator ausrüsten, der den Betrieb des Inkubators für mindestens 30 Minuten sicherstellt. Der Patient wird dadurch auch beim Wechsel seines Standortes nur für ganz kurze Zeit nicht mit geheizter Frischluft versehen.

In vielen Fällen muß der Patient nicht nur mit Luft, sondern auch mit Luft versorgt werden, die mit Sauerstoff angereichert ist. Auch eine Anreicherung der Luft mit reinem Sauerstoff ist möglich.

Bei einer vorteilhaften Ausführungsform ist nicht nur vorgesehen, daß die Lufttemperatur geregelt wird, sondern daß auch die Luftfeuchtigkeit geregelt wird.

Die Meßspule für die Kernspintomographie könnte im Tomographen angeordnet sein, so daß der Inkubator in diese Meßspule hineingeschoben werden muß. Bei einer besonders zweckmäßigen Ausführungsform ist die Meßspule für die Kernspintomographie aber im Patientenraum angeordnet. Bei einer zweckmäßigen Ausführungsform kann die Meßspule relativ zum Patienten verstellbar sein, so daß verschiedene Körperteile des Patienten untersucht werden können.

Sauerstoffsensoren stören die Bildgebung. Daher ist für die Überwachung des Patienten eine SpO₂-Messung möglich. Zweckmäßigerweise ist der Inkubator mikroprozessorgesteuert.

Zweckmäßigerweise sind an den Stirnseiten des Patientenraumes Durchführungen für Beatmungs-, Narkose-, Infusionsschläuche und/oder andere Schläuche und für das Signalkabel der Meßspule vorgesehen.

Um zu verhindern, daß die Elektronik und die Motoren durch das Magnetfeld gestört werden oder aber diese elektrischen und elektronischen Bauteile die Messung stören, ist die Elektronik auf geeignete Weise abgeschirmt. Die Spannungsversorgung für die Elektronik findet mit einem speziell abgeschirmten Schaltnetzteil statt, während die Leistungselemente (Heizung, Anfeuchter) über Halbleiterrelais mit Hilfe von Pulspausenmodulation direkt mit Netzspannung versorgt werden. Mit Hilfe eines Echtzeitbetriebssystems und einem speziellen Softwareprogramm wird der gesamte Inkubator geregelt und bei Abweichungen von den Sollwerten entsprechend optisch und/oder akustisch Alarm gegeben. Die Abschirmung und der mechanische Aufbau des Gerätes sind so gestaltet, daß der Inkubator durch entsprechende Varianten sowohl für offene als auch geschlossene Tomographen mit Magnetfeldern von 0,2 bis 3 Tesla geeignet ist. Außerdem ist der Inkubator so ausgelegt, daß er mit zwei Personen aus dem Tomographenraum getragen und im Vorraum auf einem entsprechenden Fahrgestell befestigt werden kann. Das Fahrgestell verfügt dabei über die elektrische Energiequelle in Form eines Akkumulators. Falls der Patient nicht mit Luft, sondern zusätzlich mit Sauerstoff versorgt werden soll, befinden sich selbstverständlich auch die entsprechenden Gasflaschen auf dem Fahrgestell.

Die Anzeige und Einstellung der Parameter erfolgt zweckmäßigerweise digital. Selbstverständlich werden auch die relevanten internationalen Normen für Medizingeräte erfüllt. Weiter wird man zweckmäßigerweise vorsehen, daß die Frischluftansaugung durch entsprechende Filter insbesondere Partikelfilter erfolgt, um eine Verunreinigung der Atemluft für den Patienten zu vermeiden. Soweit dies irgendwie möglich ist, wird man nicht nur für den Inkubator, sondern auch für Zubehörteile nach Möglichkeit kein Metall verwenden. Das Fahrgestell wird vorteilhafterweise ebenfalls aus magnetisch nicht anziehbarem Material bestehen. Durchführungen und Halterungen für Hilfsgeräte wie z.B. Infusionspumpen können ebenfalls beim erfindungsgemäßen Inkubator vorgesehen sein.

Die Erfindung wird im folgenden anhand von vorteilhaften Ausführungsformen unter Bezugnahme auf die beigefügten Zeichnungen beschrieben. Es zeigen:
- Fig. 1: in der perspektivischen Ansicht eine erste Ausführungsform des Inkubators der Erfindung;
- Fig. 2: in schematischer Darstellung die Anordnung der verschiedenen Komponenten des Inkubators der Erfindung;
- Fig. 3: einen Längsschnitt durch eine zweite Ausführungsform des Inkubators der Erfindung;
- Fig. 4: einen Querschnitt entlang der Linie IV-IV von Fig. 3;
- Fig. 5: einen Querschnitt entlang der Linie V-V von Fig. 3; und
- Fig. 6: einen Querschnitt entlang der Linie VI-VI von Fig. 3.

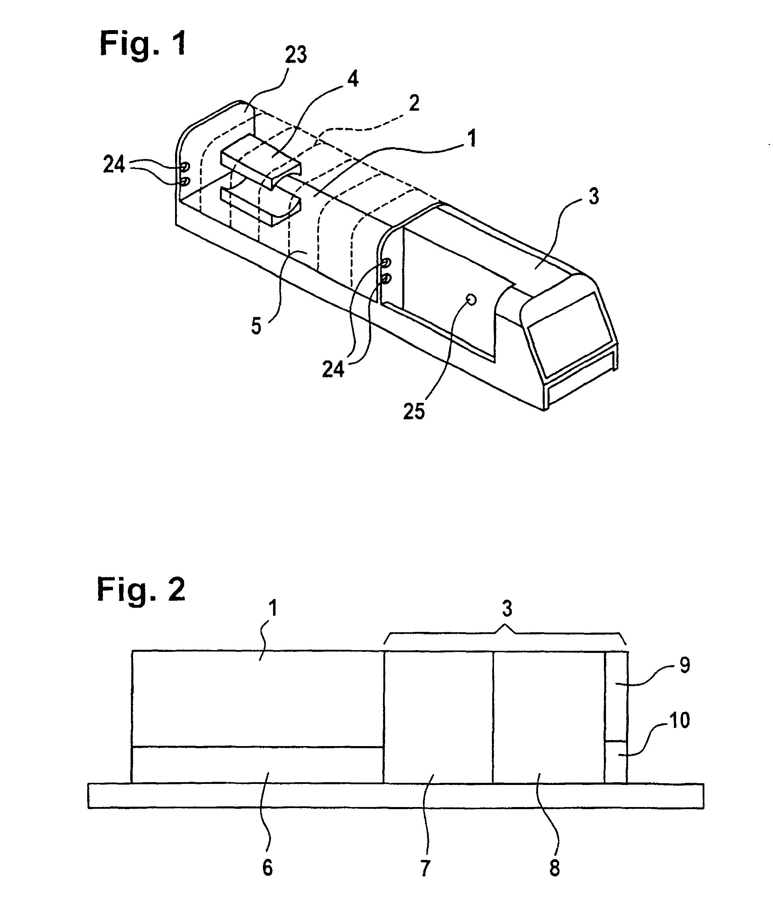

Der in Fig. 1 gezeigte Inkubator der Erfindung weist einen Aufnahmeraum 1 für den Patienten auf, der mit einer gestrichelt gezeichneten Haube 2 verschlossen werden kann. An einem Ende des Aufnahmeraums 1 für den Patienten befindet sich der Versorgungs- und Steuerteil 3. Im Patientenraum 1 ist auch eine Spule 4 zum Aufnehmen der Signale für die Kernspintomographie vorgesehen. Der Patient liegt auf einer Liegefläche 5.

In Fig. 2 ist schematisch der Aufbau des Inkubators und die Anordnung der einzelnen Komponenten gezeigt. Unterhalb des Aufnahmeraums 1 befindet sich eine Luftführung 6, die von einem Teil 7 mit Luft versorgt wird. Im Teil 8 befindet sich die elektrische Versorgung und Steuerung. Teil 9 ist ein Bedienungsfeld, am Teil 10 sind die Anschlüsse für elektrische Stromversorgung und falls erforderlich für Sauerstoff vorgesehen.

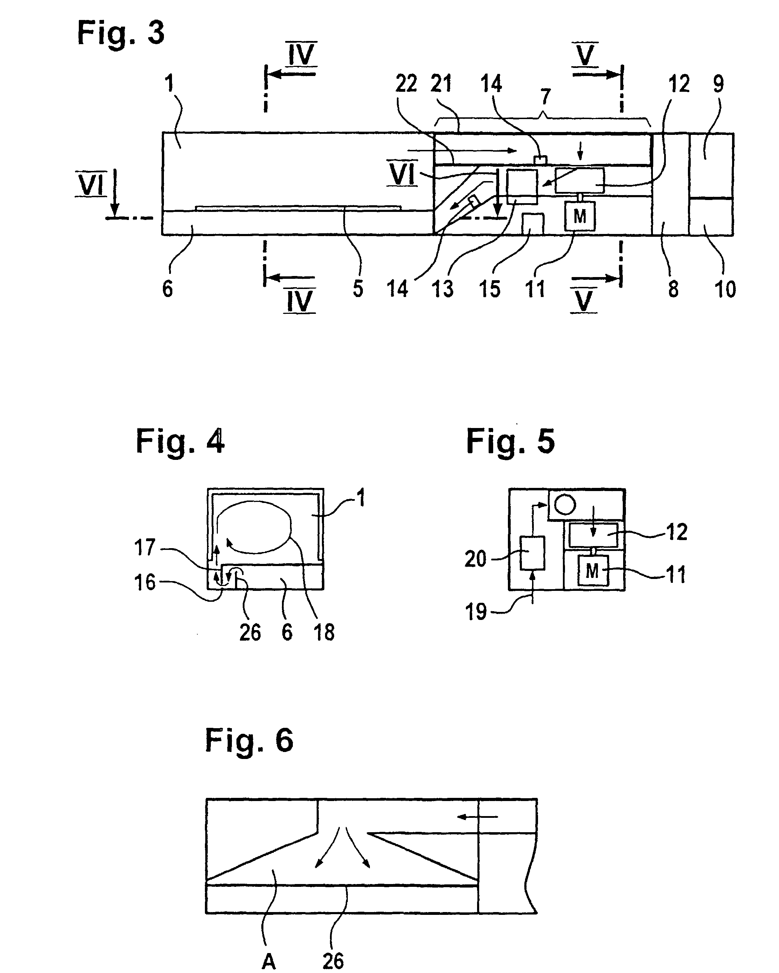

Die Fig. 3 bis 5 zeigen eine etwas andere Ausführungsform, die sich von der Ausführungsform der Fig. 1 in erster Linie durch die geometrische Außenform des Inkubators unterscheidet. Mit einem Motor 11 wird ein Lüfter 12 betrieben, der für Luftzirkulation in Richtung der Pfeile sorgt. Die Luft wird dabei über eine Heiz/Befeuchtungseinrichtung 13 und einen Fühler 14 geleitet, mit dem Temperatur und Luftfeuchtigkeit gemessen werden. Aufgrund der entsprechenden Signale erfolgt dann mit Hilfe eines Mikroprozessors 15 die Regelung des Inkubators. Die Luft, die umgewälzt wird, wird in den Beruhigungsraum A unter der Liegefläche 5 geblasen (Fig. 3 und 6) wie in Fig. 4 gezeigt ist, seitlich über eine entsprechende Wand 17 in den Patientenraum. Durch den Spalt 16 unter der Wand 17 wird der Überdruck des Beruhigungsraums in die notwendige Strömungsgeschwindigkeit für die Ausbildung der walzenförmigen Strömung 18 erzeugt. Mit 26 ist eine Umlenkwand bezeichnet. Anschließend gelangt die Luft dann wieder in den Luftführungsteil 7 und wird erneut eingeblasen. Wie dies durch den Pfeil 19 in Fig. 5 gezeigt ist, wird über ein Filter 20 laufend Frischluft angesaugt bzw. bei Bedarf Sauerstoff hinzugefügt, um die Luft zu erneuern, während die verbrauchte Luft durch nicht gezeigte Öffnungen, z.B. im Bereich der Abdeckung 4 entweicht, die aus durchsichtigem Kunststoff besteht. Die Stromversorgung erfolgt über das in Fig. 3 nur schematisch dargestellte Teil 8, während im Teil 9 ein Bedienungsfeld und im Teil 10 die Anschlüsse für elektrische Energie und ggf. Gas vorgesehen sind.

Die Spule kann entweder fest eingebaut sein z. B. für Kopfuntersuchungen oder bei speziellen Spulen und Untersuchungen manuell positioniert werden. Außerdem können in den Stirnwänden 23 bei 24 angeordnete Durchführungen für Infusionsschläuche, Narkoseschläuche und Meßkabel vorgesehen sein. Bei 25 kann der SpO₂ Sensor (Kabel und Sensor nicht dargestellt) angeschlossen und zum Patienten geführt werden. Die Messung funktioniert über Lichtwellenleiter zum Ausschluß von Störungen für den Kernspintomographen.

## Patentansprüche

1. Inkubator für neu- und frühgeborene Patienten, der in das Magnetfeld eines Kernspintomographen einführbar ist, mit Einrichtungen (6, 7, 11, 12, 20) für Frischluftzufuhr und -zirkulation und Antrieb dieser Einrichtungen und mit Einrichtungen (4, 7, 8, 9, 10, 11, 12, 13, 14, 15) zum Heizen und Befeuchten der Luft, zum Regeln der Temperatur derselben und zum Untersuchen des Patienten, **dadurch gekennzeichnet, daß** die Einrichtungen (7, 8, 9, 10, 11) elektrisch angetrieben am Inkubator an der, der stelle für die spule für Kopfuntersuchungen gegenüberliegenden Stirnseite des Patientenraums (1) angebracht sind und im Patientenraum (1) die Lufteinströmung an einer Längsseite erfolgt, so daß sich eine walzenförmige Luftbewegung (18) im Patientenraum ausbildet und die Absaugung an einer Stirnseite in der Mitte der Luftwalze (18) erfolgt.

2. Inkubator nach Anspruch 1, **dadurch gekennzeichnet, daß** die Luftzufuhr durch einen Kanal (6) unterhalb der Liegefläche (5) für die Patienten erfolgt.

3. Inkubator nach Anspruch 1 oder 2, **dadurch gekennzeichnet, daß** er Einrichtungen (20) zum Zuführen von Sauerstoff aufweist.

4. Inkubator nach einem der Ansprüche 1 bis 3, **dadurch gekennzeichnet, daß** er Einrichtungen (14, 15) zum Regeln der Feuchtigkeit der dem Patienten zugeführten Luft aufweist.

5. Inkubator nach einem der Ansprüche 1 bis 4, **dadurch gekennzeichnet, daß** die Meßspule (4) für die Kernspintomographie im Patientenraum angeordnet ist.

6. Inkubator nach Anspruch 5, **dadurch gekennzeichnet, daß** die Meßspule (4) relativ zum Patienten verstellbar ist.

7. Inkubator nach einem der Ansprüche 1 bis 6, **dadurch gekennzeichnet, daß** er Einrichtungen (25) zur Sauerstoffsättigungsmessung aufweist.

8. Inkubator nach einem der Ansprüche 1-7, **dadurch gekennzeichnet, daß** er Einrichtungen zum Regeln der Lufttemperatur aufweist.

9. Inkubator nach einem der Ansprüche 1-8, **dadurch gekennzeichnet, daß** die Luftumwälzung mittels eines Elektromotors (11) und eines Ventilators (12) erfolgt.

10. Inkubator nach einem der Ansprüche 1 bis 9, **dadurch gekennzeichnet, daß** er mikroprozessorgesteuert ist.

11. Inkubator nach einem der Ansprüche 1 bis 10, **dadurch gekennzeichnet, daß** an der Stirnseite des Patientenraumes Durchführungen (24) für Beatmungs-, Narkose-, Infusionsschläuche und andere Schläuche und für das Signalkabel der Meßspule (4) vorgesehen sind.

12. Inkubator nach einem der Ansprüche 1 bis 11, **dadurch gekennzeichnet, daß** der Patientenraum (1) eine transparente Haube (2) zur Beobachtung des Patienten aufweist.

13. Inkubator nach einem der Ansprüche 1-12, **dadurch gekennzeichnet, daß** der Bedienteil (9) digitale Anzeigen und Drehknöpfe zum Verstellen der Sollwerte aufweist.

## Claims

1. Incubator for new born and premature patients that can be introduced into the magnetic field of a nuclear spin tomograph, with facilities (6, 7, 11, 12, 20) for supplying and circulating fresh air and driving these facilities and with facilities (4, 7, 8, 9, 10, 11, 12, 13, 14, 15) for heating and humidifying the air, for controlling the temperature thereof and for examining the patient, **characterised in that** the facilities (7, 8, 9, 10, 11) are installed, electrically driven, on the incubator at the end face of the patient chamber (1) opposite the location for the coil for head examination and the air inflow into the patient chamber (1) takes place on one longitudinal side, so that a cylindrical air movement (18) develops in the patient chamber and the extraction takes place at an end face in the centre of the air cylinder (18).

2. Incubator according to Claim 1, **characterised in that** the air is supplied through a duct (6) below the surface (5) on which the patient lies.

3. Incubator according to Claim 1 or 2, **characterised in that** it has facilities (20) for supplying oxygen.

4. Incubator according to one of Claims 1 to 3, **characterised in that** it has facilities (14, 15) for controlling the humidity of the air supplied to the patient.

5. Incubator according to one of Claims 1 to 4, **characterised in that** the exploring coil (4) for the nuclear spin tomography is arranged in the patient chamber.

6. Incubator according to Claim 5, **characterised in that** the exploring coil (4) is adjustable relative to the patient.

7. Incubator according to one of Claims 1 to 6, **characterised in that** it has facilities (25) for oxygen saturation measurement.

8. Incubator according to one of Claims 1 - 7, **characterised in that** it has facilities for controlling the air temperature.

9. Incubator according to one of Claims 1 - 8, **characterised in that** the air circulation is effected by means of an electric motor (11) and fan (12).

10. Incubator according to one of Claims 1 to 9, **characterised in that** it is controlled by a microprocessor.

11. Incubator according to one of Claims 1 to 10, **characterised in that** openings (24) for resuscitation, anaesthetic and infusion tubes and other tubes and for the signal cable for the exploring coil (4) are provided in the end face of the patient chamber.

12. Incubator according to one of Claims 1 to 11, **characterised in that** the patient chamber (1) has a transparent hood (2) for observing the patient.

13. Incubator according to one of Claims 1 - 12, **characterised in that** the control part (9) has digital displays and rotary knobs for adjusting the set values.

## Revendications

1. Incubateur pour patients nouveaux-nés et prématurés, qui peut être introduit dans le champ magnétique d'un appareil pour résonance magnétique nucléaire, comportant des dispositifs (6, 7, 11, 12, 20) pour l'arrivée et la circulation d'air frais et l'entraînement de ces dispositifs, et comportant des dispositifs (4, 7, 8, 9, 10, 11, 12, 13, 14, 15) pour chauffer et humidifier l'air, pour réguler la température de celui-ci et pour examiner les patients, **caractérisé en ce que** les dispositifs (7, 8, 9, 10, 11), entraînés électriquement, sont placés sur l'incubateur sur le côté frontal, opposé à l'emplacement pour la bobine pour examen de la tête, de l'enceinte (1) du patient et dans l'enceinte (1) du patient, l'arrivée d'air s'effectue sur un côté longitudinal, de sorte qu'il se produit un mouvement d'air (18) en forme de rouleau dans l'enceinte du patient et l'aspiration se produit sur un côté frontal au milieu du rouleau d'air (18).

2. Incubateur selon la revendication 1, **caractérisé en ce que** l'arrivée d'air s'effectue à travers un canal (6) au-dessous de la surface de couchage (5) des patients.

3. Incubateur selon la revendication 1 ou 2, **caractérisé en ce qu'**il comporte des dispositifs (20) pour l'alimentation en oxygène.

4. Incubateur selon l'une des revendications 1 à 3, **caractérisé en ce qu'**il comporte des dispositifs (14, 15) pour réguler l'humidité de l'air amené au patient.

5. Incubateur selon l'une des revendications 1 à 4, **caractérisé en ce que** la bobine de mesure (4) pour la résonance magnétique nucléaire est disposée dans l'enceinte du patient.

6. Incubateur selon la revendication 5, **caractérisé en ce que** la bobine de mesure (4) est réglable par rapport au patient.

7. Incubateur selon l'une des revendications 1 à 6, **caractérisé en ce qu'**il comporte des dispositifs (25) pour la mesure de la saturation en oxygène.

8. Incubateur selon l'une des revendications 1 à 7, **caractérisé en ce qu'**il comporte des dispositifs pour la régulation de la température de l'air.

9. Incubateur selon l'une des revendications 1 à 8, **caractérisé en ce que** le brassage de l'air s'effectue au moyen d'un moteur électrique (11) et d'un ventilateur (12).

10. Incubateur selon l'une des revendications 1 à 9, **caractérisé en ce qu'**il est commandé par un microprocesseur.

11. Incubateur selon l'une des revendications 1 à 10, **caractérisé en ce que** sur le côté frontal de l'enceinte du patient sont prévues des traversées (24) pour des tubes de respiration, d'anesthésie et de perfusion ainsi que d'autres tuyaux et pour le câble de signalisation de la bobine de mesure (4).

12. Incubateur selon l'une des revendications 1 à 11, **caractérisé en ce que** l'enceinte (1) du patient comporte un capot (2) transparent pour l'observation du patient.

13. Incubateur selon l'une des revendications 1 à 12, **caractérisé en ce que** la partie de commande (9) comporte des affichages numériques et des boutons tournants pour le réglage des valeurs de consigne.
